# NOUVEAU FASCICULE DE BREVET EUROPEEN

(11) **EP 0 882 155 B2**
(45) Date de publication et mention de la décision concernant l'opposition: **09.11.2005**
(45) Mention de la délivrance du brevet: 19.12.2001
(21) Numéro de dépôt: 97903432.9
(22) Date de dépôt: 10.02.1997
(51) Int. Cl.: D21H 17/07, D21H 17/06, D21H 21/24, D21H 17/60

(54) **COMPOSITION DE LOTION ADOUCISSANTE, UTILISATION EN PAPETERIE ET PRODUIT PAPETIER OBTENU**
WEICHMACHERLOTIONZUSAMMENSETZUNG, IHRE VERWENDUNG IN DER PAPIERHERSTELLUNG UND PAPIERPRODUKT
SOFTENING LOTION COMPOSITION, USE THEREOF IN PAPER MAKING, AND RESULTING PAPER PRODUCT

(30) Priorité: 19.02.1996 FR 9602023
(43) Date de publication de la demande: 09.12.1998
(73) Titulaire: Georgia-Pacific France, 68320 Kunheim (FR)
(72) Inventeur: BRET, Bruno, F-68000 Colmar (FR); LEBOEUF Jean-François, F-68180 Horbourgwihr (FR)
(74) Mandataire: Cortier, Sophie
(86) Numéro de dépôt international: PCT/FR1997/000256
(87) Numéro de publication internationale: WO 1997/030217

(56) Documents cités:
- EP-A- 0 122 141
- WO-A-95/35412
- GB-A- 338 224
- US-A- 3 554 862
- US-A- 5 415 737
- Hawley:"Condensed Chemical Dictionary", 11ème édition

## Description

L'invention concerne généralement une nouvelle composition pour une lotion adoucissante destinée au traitement d'un produit papetier absorbant tel qu'une feuille en ouate de cellulose. Cette lotion est appliquée ou imprégnée sur au moins une surface du produit et donne au papier un toucher doux, glissant, tout en étant sec, c'est à dire non graisseux ou huileux. L'invention concerne également les produits papetiers absorbants dont au moins une surface est imprégnée d'une telle lotion.

L'invention trouve application dans la fabrication des produits papetiers tels que les papiers à usage domestique ou sanitaire. On peut citer plus particulièrement les papiers dont l'utilisation implique un contact direct avec la peau et des frottements répétés avec celle-ci, tels que les mouchoirs en papier jetables, le papier toilette ou encore tout autre produit en papier destiné à essuyer la peau, serviette à démaquiller, lingettes sèches, etc.

Les personnes atteintes de rhume, grippe ou différentes allergies provoquant l'écoulement nasal, sont amenées à se moucher très fréquemment. Elles ont souvent le nez irrité et rouge, dû à une hypersensibilisation de la peau par cet écoulement nasal. Pour des raisons pratiques, elles utilisent des mouchoirs en papier classiques que l'on trouve dans le commerce sous la forme de mouchoirs en boîte, encore dénommés mouchoirs "facial", ou mouchoirs pliés, disposés dans des étuis. Après quelques essuyages successifs du nez avec ces mouchoirs, la peau dans la région située au niveau et autour du nez, devient de plus en plus irritée, enflammée et même douloureuse. Il est par conséquent nécessaire d'adoucir la surface de ces mouchoirs afin de limiter, voire supprimer tout phénomène d'irritation provoqué par le frottement de la surface du mouchoir sur la peau. L'idéal serait d'atteindre l'impression de douceur que procure un mouchoir en tissu, venant d'être lavé et repassé.

Dans un autre domaine qu'est le papier toilette, les mêmes exigences de douceur sont requises pour des contacts répétés avec la peau sous une action simultanée de frottement. En particulier dans le cas des personnes souffrant d'irritations de la peau dans la région anale ou d'hémorroïdes, l'utilisation d'un papier toilette présentant un toucher parfois un peu rugueux ne fait qu'irriter davantage la peau lorsque le papier est appliqué sous pression sur la peau.

On a donc cherché à adoucir de manière générale les produits ou feuilles en papier tels que les feuilles en ouate de cellulose par différents moyens, mécaniques ou chimiques.

En ce qui concerne les moyens mécaniques, des techniques ont été développées pour améliorer plus particulièrement l'aspect et l'état de surface de la feuille en papier en lui donnant un toucher plus glissant. Dans le cas des mouchoirs, la feuille est par exemple calandrée pour aplanir les crêtes formées au cours du crêpage de la feuille. On peut encore traiter la surface de la feuille par friction pour éliminer toutes les aspérités. Mais ces moyens se révèlent le plus souvent insuffisants. Le brevet européen n° 0 029 269 décrit un procédé particulier de fabrication de la feuille dans lequel la nature des suspensions de fibres formant les différentes couches de la feuille et l'association de ces couches entre-elles sont importantes pour le toucher velouteux souhaité. Mais ce procédé limite le choix dans la fourniture des fibres et entraîne des contraintes dans les premières étapes de la phase humide du procédé de fabrication.

Par les moyens chimiques, on entend toute composition adoucissante à base d'un ou plusieurs composés chimiques. Deux catégories de compositions adoucissantes peuvent être distinguées : les additifs ou compositions adoucissantes qui sont incorporés directement dans la pâte ou composition de fabrication ou encore appliquées sur une feuille de papier humide et les compositions adoucissantes ou lotions qui sont appliquées en surface d'un produit ou d'une feuille de papier, à l'état sec, c'est à dire qui a été préalablement séché.

Dans le premier cas, ces additifs sont généralement utilisés comme agent déliant pour les fibres et permettent de ce fait d'assouplir la feuille ainsi obtenue. De nombreux brevets ont été déposés dans ce domaine, on peut citer à titre d'exemple, EP -A-0 049 924, EP-B-0 347 176, US 2 944 931, US 5 415 737 et WO 95/10661.

EP-A-0 049 924 décrit l'incorporation, dans la composition de fabrication, d'un composé ammonium quaternaire et d'au moins un tensioactif non-ionique sélectionné parmi les dérivés d'oxyde d'éthylène d'alcools gras et d'acides gras pour la fabrication d'un papier absorbant doux. EP-B-0 347 176 a pour objet un papier en ouate de cellulose comprenant au moins un tensioactif non cationique, ce dernier étant appliqué sur une feuille de papier humide. Mais ce tensioactif peut migrer à l'intérieur de la feuille et complètement revêtir les fibres entraînant un effet déliant sur les fibres qui conduit à une diminution de la résistance à la traction. US 2 944 931 révèle un procédé pour améliorer la douceur du papier sanitaire et son toucher, consistant à ajouter à la composition de fabrication une émulsion stable aqueuse comprenant de 1 à 90 % en poids de lanoline et de 10 à 99 % en poids d'un émulsifiant cationique tel que des sels d'ammonium quaternaire. US 5 415 737 concerne un produit fini en papier, doux, comprenant un composé ester ammonium quaternaire à base d'huile végétale, ce composé étant également ajouté dans la composition de fabrication. WO 95/10661 enseigne un procédé pour fabriquer un papier doux, ayant un toucher amélioré, consistant à ajouter des sels d'ester d'acide gras de triéthanol amine quaternaire, comme adoucissants dans les suspensions aqueuses de fibres. Mais, généralement, pour l'ensemble de ces brevets, la surface du produit ou de la feuille ne présente pas l'aspect glissant recherché. C'est le produit ou la feuille dans son ensemble qui est plus souple. Par ailleurs, les pertes en composition adoucissante au cours du procédé de fabrication de la feuille ne sont pas négligeables.

Dans le même esprit, le brevet américain N° 5 279 767 décrit plus précisément une composition adoucissante comprenant un mélange d'un composé ammonium quaternaire et un composé polyhydroxy. Cette composition est préparée en mélangeant dans un premier temps ces deux composés à une température à laquelle ils sont miscibles, puis à diluer le mélange dans l'eau à température élevée, de manière à former une dispersion aqueuse de vésicules (ou encore micelles). Cette composition est incorporée de préférence dans la composition de fabrication et peut éventuellement être appliquée en surface de la feuille formée, à l'état humide, avant séchage. Dans ce brevet, on pense que les vésicules se brisent au moment du séchage. La majorité du composé polyhydroxy ainsi "libéré" pénètre à l'intérieur des fibres cellulosiques et améliore leur souplesse, et l'autre partie est retenue en surface des fibres permettant d'augmenter la vitesse d'absorption des fibres. En raison des liaisons ioniques, le composé ammonium quaternaire reste à la surface des fibres cellulosiques permettant d'améliorer l'impression au toucher et la douceur du produit. Ce brevet ne mentionne pas de toucher glissant malgré une douceur améliorée. Ce type de composition est prévu pour augmenter la souplesse des fibres, elle agit essentiellement dans la structure interne de la feuille et non pas directement et essentiellement en surface d'une feuille. Des variantes de cette composition sont décrites dans d'autres brevets tels que par exemple, WO 94/29520 et WO 94/29521.

Dans le second cas, les compositions adoucissantes sont destinées à être appliquées directement en surface du produit ou d'une feuille de papier absorbant qui a été préalablement séché. Elles ont pour principale fonction d'être émolliente pour la peau.

De nombreux brevets illustrent également ce type de lotion.

On peut citer par exemple pour le papier toilette ou des serviettes d'essuyage destinées à un usage de proctologie, le brevet américain N° 3 264 188 ou encore le brevet français N° 2 376 650 décrivant des lotions procurant un toucher gras. Ce dernier décrit un produit en papier pour essuyer la peau, traité avec un émollient lipophile et nettoyant, la composition étant essentiellement non polaire et non aqueuse. Cet émollient peut être une huile minérale, du petrolatum, des cires de paraffine, des acides gras, des alcools gras, des esters d'acide gras, des dérivés de glycérides, de la lanoline, des polysiloxanes, etc. L'émollient se dépose sur la surface de la peau et forme un film mince. Il permet le nettoyage de la peau par l'enlèvement des souillures. On peut également mentionner le brevet américain n° 4 481 243 ayant pour objet une feuille composée de deux plis. Un émollient procurant un toucher gras, est distribué sur une grande partie de la surface de la feuille. Mais l'émollient n'est pas appliqué dans une zone où l'on associe les plis par gaufrage.

Des huiles de silicone telles que les polysiloxanes peuvent être appliquées sur une feuille en ouate de cellulose comme le révèlent les brevets européens N° 0 347 153, N° 0 595 994, et la demande de brevet européen N° 0 656 971. Mais certaines huiles de silicone sont hydrophobes et diminuent la mouillabilité en surface du papier ainsi traité.

Le brevet américain N° 3 305 392 a pour objet une feuille de papier sur laquelle a été appliquée un émollient, par déplacement de la feuille sur un bloc relativement solide d'une composition émolliente ressemblant à de la cire. Cette composition comprend une partie lubrifiante et adoucissante, telle que du stéarate de zinc, du stéarate d'aluminium, de sodium, de calcium, ou de magnésium, de l'acide stéarique, des esters d'acide palmitique, spermacétique, de l'alcool stéarylique, avec en plus éventuellement des esters polyéthylène glycol d'acide laurique et stéarique comme lubrifiants efficaces. Des composés peuvent être ajoutés tels que de l'acide oléique, de l'huile minérale, du tallow glycéride, du distéaryl méthyl amine, des amines grasses primaires et secondaires et des dérivés de lanoline, permettant de donner une forme plastique à la composition. Afin de réduire la migration de composés à l'intérieur de la feuille, on peut encore prévoir des agents qui possèdent un groupement actif se fixant sur les fibres de cellulose, ces agents sont cationiques. Etant donné que ce type de composition est sous une forme relativement solide, son application ne peut se faire qu'à vitesse réduite et les quantités appliquées ne sont pas optimisées par une telle technique.

D'autres brevets concernent également des lotions solides ou semi-solides à température ambiante. Le brevet américain N° 3 896 807 décrit une composition émolliente sous la forme d'un solide non collant et non huileux. Cette composition est chauffée ou mélangée à des solvants non aqueux du type acétone, chloroforme trichloroéthylène, xylène, xylol et autres solvants aromatiques, pour être imprégnée sous une forme liquide sur un substrat par exemple en papier. Cette composition nécessite donc, pour être appliquée, soit des moyens de chauffage, soit des solvants pour la plupart inutilisables d'un point de vue toxicologique. Les composants essentiels de cette composition sont une phase huileuse contenant un matériau huileux tel qu'une huile minérale, du petrolatum, de la paraffine, de l'huile végétale et différentes huiles animales, et éventuellement des émollients tels que l'alcool cétylique, le propylène glycol, la glycérine, le triéthylène glycol, et des cirres, et un agent émulsifiant. Ce type de lotion est intéressant car au contact de l'humidité de la peau, la composition forme une émulsion huile dans eau, qui agit comme émollient.

Des demandes internationales de brevet plus récentes, WO 95/16824 et WO 95/35412, proposent encore une lotion solide ou semi-solide à 20° C, exempte d'eau, mais présentant des contraintes au niveau de son procédé d'application sur la feuille. En effet, ce procédé implique des moyens de chauffage avec tous les problèmes que cela engendre tant sur le plan du matériel choisi pour l'imprégnation, que sur l'état liquide et stable de la lotion qui doit rester à température relativement constante dans ce procédé d'application.

Certains composés émollients tels que la lanoline présentent des inconvénients liés à leur odeur ou leur effet sur la diminution de l'absorption de la feuille. Le brevet européen N° 0 365 726 tente de remédier à ces problèmes en proposant des lotions comprenant un seul composant soluble dans l'eau : le lauroamphoglycinate, des dérivés homo- ou copolymère ammonium quaternaire, un complexe phospholipidique triquaternaire ou du glucose glutamate.

La demande de brevet français N° 2 538 238 décrit un procédé consistant à faire passer un substrat, par exemple des bandes de papier destinées à former des serviettes d'essuyage, dans une lotion dissoute dans un solvant organique et à évaporer ce solvant. On peut également imprégner jusqu'à pratiquement saturation le substrat, d'une émulsion aqueuse dont les ingrédients seront absorbés par le substrat puis sécher pour éliminer complètement l'eau de l'émulsion. La lotion comprend un composé tensioactif et un corps gras. Les deux variantes de procédé mentionnées précédemment impliquent des étapes ultérieures d'évaporation ou de séchage que l'on préfère éviter dans la fabrication des feuilles en ouate de cellulose.

Certaines lotions, une fois appliquées, modifient les propriétés physiques et mécaniques de la feuille de papier, telles que la capacité d'absorption, la résistance à la traction dans le sens marche et dans le sens travers, etc. Il est particulièrement important pour la feuille en papier imprégnée d'une lotion de garder d'aussi bonnes propriétés de résistance que la même feuille sur laquelle aucune lotion n'aurait été appliquée.

L'invention a pour but de pallier l'ensemble des inconvénients rencontrés lors de l'utilisation de lotions appliquées sur un produit papetier absorbant, aussi bien au niveau du procédé d'application de la lotion sur la surface d'un produit qu'au niveau de l'utilisation du produit dans ces différentes fonctions d'essuyage de la peau.

L'invention a pour but de fournir une composition pour une lotion donnant un toucher particulièrement doux et glissant au papier, limitant les phénomènes d'irritation entraînés par les frottements du produit en papier sur la peau. L'invention a pour autres buts de fournir une composition procurant au papier un toucher plus moelleux et une plus grande souplesse. En outre, ce toucher reste sec, à la différence de certaines lotions qui, une fois imprégnées sur un produit en papier, laissent un toucher gras, un film gras se déposant sur la peau ou sur des lunettes occasionnellement essuyées avec ce type de produit, mouchoir ou serviette à démaquiller.

L'invention a pour but de fournir une composition pour une lotion qui est liquide à une température d'au moins environ 5°C. De préférence, la composition est liquide à une température comprise dans l'intervalle d'environ 10 à environ 40°C, supprimant ainsi toutes les difficultés d'application des lotions solides ou semi-solides à température ambiante qu'il faut, pour la plupart, chauffer afin de les appliquer sur la surface d'un produit ou d'une feuille en papier.

L'invention a encore pour but d'appliquer en faibles quantités une lotion en surface de produits papetiers.

L'invention a également pour but de fournir un produit papetier absorbant dont au moins une surface a été imprégnée d'une telle lotion, et tous les produits en papier en découlant.

Les propriétés physiques et mécaniques du produit papetier absorbant selon l'invention ainsi traité, ne sont pas substantiellement modifiées, tant son épaisseur que sa capacité d'absorption, ou sa résistance à la traction dans le sens marche et travers. Un produit comportant la lotion peut être avantageusement gaufrée sans que cela présente un quelconque problème.

Dans la description qui suit, on entend par produit papetier absorbant, une feuille comprenant essentiellement des fibres papetières, destinée à la fabrication de produits en papier à usage domestique et sanitaire ou le produit papetier absorbant fini, en tant que tel. Cette feuille en papier peut être une feuille en ouate de cellulose ou tissu ouaté, une feuille en papier absorbant de faible grammage obtenue par exemple par un procédé par soufflage traversant, une feuille ou nappe formée par voie sèche, constituée de fibres papetières liées par un liant thermoplastique tel qu'un latex, ou encore une feuille en papier absorbant constituée de fibres papetières en majeure partie et de fibres synthétiques, ou tout produit papetier équivalent. La feuille peut donc être crêpée ou non et calandrée ou non. Elle est formée d'un ou plusieurs plis. D'autres caractéristiques relatives notamment au grammage seront données dans la description qui suit.

L'invention a pour objet une composition pour une lotion adoucissante, liquide à une température d'au moins 5°C, destinée au traitement d'un produit papetier absorbant.

Selon une caractéristique essentielle de l'invention, la composition comprend :
a) de 1 à 10 pour cent en poids d'un composant contenant essentiellement un composé ammonium quaternaire,
b) de 5 à 99 pour cent en poids d'un composant aqueux émollient comprenant comme matière active :
   i) un ou plusieurs alcools gras linéaires, saturés, ayant au moins 16 atomes de carbone, et
   ii) un ou plusieurs esters cireux ayant au moins au total 24 atomes de carbone, et éventuellement
   iii) un ou plusieurs agents émulsifiants non-ioniques et/ou amphotères, et
c) q.s.p. 100 pour cent en poids d'un solvant du type polyol, huile minérale ou leurs mélanges.

Selon une autre caractéristique de l'invention, le composant aqueux émollient précité est une dispersion aqueuse.

Selon une caractéristique avantageuse de l'invention, le composant aqueux émollient précité comprend de 15 à 45 pour cent en poids de matières actives et de 55 à 85 pour cent en poids d'eau et de préférence, de 20 à 40 pour cent en poids de matières actives et de 60 à 80 pour cent en poids d'eau.

Selon encore une autre caractéristique de l'invention, le composant aqueux émollient précité comprend en poids de matières actives :
i) 35 à 90 % d'alcools gras linéaires saturés ayant de 18 à 24 atomes de carbone,
ii) 1 à 50 % d'esters cireux ayant au total de 24 à 48 atomes de carbone,
iii) 0 à 20 % d'agents émulsifiants non-ioniques et/ou amphotères, et
iv) 0 à 50 % de cire ou huile minérale,
le total des quantités en composants s'élevant à environ 100 % en poids de matières actives.

Selon une caractéristique préférée de l'invention, la composition comprend :
a) de 1 à 6 pour cent en poids du composant contenant essentiellement un composé ammonium quaternaire,
b) de 10 à 60 pour cent en poids du composant aqueux émollient, et
c) q.s.p. 100 pour cent en poids du solvant.

Selon une autre caractéristique préférée de l'invention, le composé ammonium quaternaire précité est un sel de formule (I) suivante : 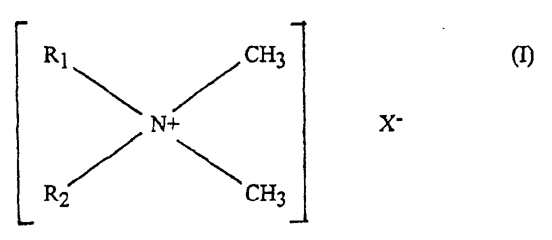 où R₁ est un groupement aliphatique ayant de préférence de 2 à 18 atomes de carbone, R₂ est un groupement aliphatique ayant de préférence de 12 à 18 atomes de carbone ou un groupement aryle, de préférence benzyle, et X est un halogène du type chlore.

L'invention a encore pour objet l'utilisation d'une lotion adoucissante dont la composition est définie ci-dessus, pour le traitement d'un produit papetier absorbant.

Selon une caractéristique essentielle de l'invention, une quantité d'environ 0,30 à environ 20 pour cent en poids sur la base du poids sec du produit, de la lotion dont la composition est définie ci-dessus, est appliquée sur le produit.

Un léger film émollient est ainsi déposé et en partie fixé à la surface du produit, ce qui permet au produit d'être doux et glissant au contact de la peau.

L'invention a aussi pour objet un produit papetier absorbant.

Selon une caractéristique essentielle de l'invention, au moins une surface du produit est imprégnée d'une lotion dont la composition est définie précédemment.

Selon une autre caractéristique essentielle de l'invention, au moins une surface du produit est imprégnée d'une lotion et ce produit comprend :
- des fibres papetières,
- au moins un composé ammonium quaternaire, et
- au moins un alcool gras linéaire, saturé, ayant au moins 16 atomes de carbone, et
- au moins un ester cireux ayant de 24 à 48 atomes de carbone, et au moins 3 pour cent de matières actives de l'ester cireux, sur la base du poids sec du produit papetier absorbant, se retrouvent en surface du produit papetier absorbant.

Selon une caractéristique avantageuse de l'invention, ce produit est un mouchoir en papier jetable.

D'autres caractéristiques et avantages de l'invention apparaîtront à la lecture de la description qui suit.

Les composants utilisés pour préparer la lotion sont les suivants :

Le composant a) contient essentiellement un composé ammonium quaternaire.

Le composé ammonium quaternaire englobe aussi bien les sels d'ammonium quaternaire que les sels d'esters ammonium quaternaire.
Les sels d'ammonium quaternaire ont la formule (I) suivante : 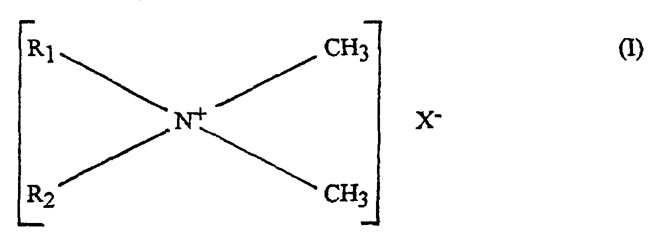 dans laquelle R₁ est un groupement aliphatique ayant de préférence de 12 à 18 atomes de carbone, R₂ est un groupement aliphatique ayant de préférence de 12 à 18 atomes de carbone ou un groupement aryle, de préférence benzyle, et X est un halogène du type chlore. Un exemple d'un tel sel est un chlorure de ditallow diméthyl ammonium quaternaire et plus précisément la forme où le groupement tallow-alkyl est hydrogéné. On peut citer par exemple le composé commercialisé sous le nom ARQUAD 2HT-75 par la société AKZO CHEMIE. Ce composé est pâteux à 25°C. On peut également envisager d'utiliser un sel d'ammonium quaternaire répondant à la définition ci-dessus, qui soit liquide à 25°C. Lorsque, pour un composé ammonium quaternaire, R₁ et R₂ dans la formule ci-dessus sont des groupements aliphatiques ayant de préférence de 12 à 18 atomes de carbone, le composant a) peut ne contenir que ce composé, seul ou en mélange avec de faibles quantités de propanol. Le composant a) peut aussi contenir un composé ammonium quaternaire qualifié d'essentiel, en mélange avec un alcool gras éthoxylé. Un exemple de ce mélange comprend un chlorure d'alkyl benzyl diméthyl ammonium et un alcool gras éthoxylé.

Les sels d'esters ammonium quaternaire ont la formule (II) suivante : 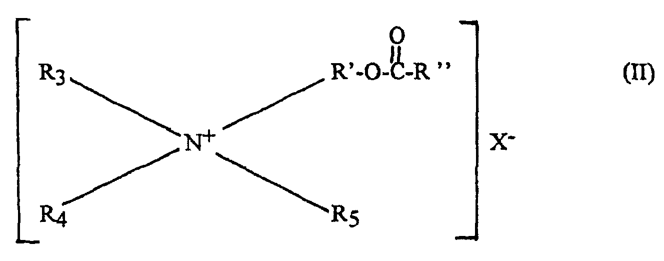 dans laquelle
R' est un groupement alkyle ayant de 1 à 6 atomes de carbone,
R" est un groupement alkyle ayant de 12 à 18 atomes de carbone,
R₃ est un groupement alkyle ayant de 1 à 6 atomes de carbone, de préférence méthyle,
R₄ est un groupement alkyle de 1 à 6 atomes de carbone, un groupement hydroxy-alkyle de 1 à 6 atomes de carbone ou un groupement

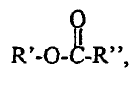

R₅ est un groupement alkyle de 1 à 6 atomes de carbone, un groupement hydroxy-alkyle de 1 à 6 atomes de carbone ou un groupement 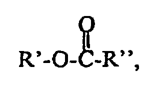 et
X⁻ est un anion, tel qu'un halogénure du type chlorure ou un méthyl sulfate.

Certains de ces composés sont avantageusement biodégradables.

A titre illustratif, on peut citer un sel ayant la formule (II) précédente dans laquelle R' est un radical éthylène, R" est un groupement alkyle ayant de 16 à 18 atomes de carbone du type « tallow » ou suif, R₃ est un groupement méthyle, R₄ est un groupement OH-CH₂-CH₂, R₅ est un groupement 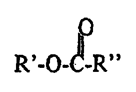 où R' et R" sont définis ci-dessus, et X⁻ est un méthyl sulfate.

Ce composé est encore dénommé di-alkyl ester de méthyl triéthanol ammonium sulfate.

D'autres sels d'esters ammonium quaternaire ayant la formule (II) précédente peuvent être préparés à partir d'acide gras tels que l'acide palmitique et l'acide stéarique.

Le composant a peut contenir un ou plusieurs composés, sels d'ester ammonium quaternaire, seuls ou en mélange avec un alcool gras et/ou un solvant du type dipropylène glycol.

Le composant a) est un agent cationique. Lorsque la lotion est appliquée sur le produit ou la feuille, cet agent a pour fonction dans la composition finale pour la lotion, de se fixer au moyen des charges positives de l'ammonium quaternaire sur les fibres de la feuille, en particulier les fibres disposées à la surface de la feuille. De ce fait, les autres composants de la lotion se trouvent "retenues" en surface de la feuille. Les composés cationiques du type ammonium quaternaire ont également pour fonction d'assouplir le produit ou la feuille lorsqu'ils sont utilisés en quantités appropriées.

Le composant aqueux émollient b) a lui-même la composition suivante. Il est à noter que chacun des constituants essentiels de ce composant sont de nature émolliente.

Le composant i) comprend un ou plusieurs alcools gras linéaires, saturés, ayant au moins 16 atomes de carbone. En général, le composant i) est un mélange d'alcools gras dont la fraction la plus importante (supérieure à 50 pour cent en poids) a des longueurs de chaîne supérieures à 16 atomes de carbone. La majorité des alcools gras a donc une longueur de chaîne se situant au-delà de 16 atomes de carbone, une faible fraction d'alcools gras pouvant se situer en deçà. Plus précisément, le composant a) est un mélange d'alcools gras ayant de 16 à 28 atomes de carbone et de préférence de 18 à 24 atomes de carbone. Encore plus préférentiellement, ces alcools gras linéaires saturés ont de 22 à 24 atomes de carbone. Des exemples d'alcools gras sont l'alcool cétylique, l'alcool stéarylique, l'alcool arachylique, l'alcool béhénique, l'alcool lignocérique et l'alcool cérylique. De préférence, le composant i) comprend l'alcool béhénique. On choisit les alcools gras d'origine naturelle, végétale ou animale, ce qui permet au composant aqueux émollient b), d'être biodégradable. Les alcools gras sont par exemple préparés à partir d'huile végétale, par transestérification, distillation, hydrogénation des esters obtenus et fractionnement des alcools gras bruts en découlant. Ces alcools gras sont qualifiés de techniques.

Pour l'application de la lotion en surface des produits papetiers, la longueur de la chaîne carbonée de l'alcool gras est essentielle pour la lotion. En effet, une longueur de chaîne suffisamment importante permettrait à ce type de molécule de rester en surface du produit papetier, par exemple une feuille de papier, et de ne pas pénétrer ou migrer à l'intérieur de ce produit.

Le composant ii) comporte un ou plusieurs esters cireux ayant au total au moins 24 atomes de carbone. Il s'agit ici également d'un mélange d'esters cireux dont la fraction la plus importante a des longueurs de chaîne supérieures à 24. De préférence, les esters cireux ont au total de 24 à 48 atomes de carbone, et sont linéaires et saturés. Plus préférentiellement, les esters cireux ont au moins au total 28 atomes de carbone. La saturation des esters permet de limiter les problèmes d'odeur liés à certains composés esters. Les esters cireux peuvent être d'origine naturelle ou synthétique.

Ils sont de préférence d'origine synthétique et dérivés d'acides gras linéaires, saturés, ayant de 6 à 24 atomes de carbone, de préférence de 10 à 24 atomes de carbone et plus préférentiellement de 12 à 22 atomes de carbone, et d'alcools gras linéaires, saturés, ayant de 6 à 24 atomes de carbone, de préférence de 10 à 24 atomes de carbone et plus préférentiellement de 12 à 22 atomes de carbone.

Ces esters cireux peuvent donc être préparés à partir d'un acide gras de longue chaîne avec un alcool gras de plus courte chaîne ou vice-versa. Les longueurs de chaîne pour l'alcool et l'acide gras peuvent encore être identiques à la condition que l'ester ait au total au moins 24 atomes de carbone. De préférence, ces esters sont issus d'un acide gras et d'un alcool gras de longueur de chaîne similaire, relativement longue : supérieure à 14.

Des exemples d'esters cireux sont les esters des acides laurique, myristique, palmitique, stéarique, arachidique, béhénique avec les alcools laurique, myristique, cétylique, stéarylique, arachylique, béhénique. On peut citer par exemple le stéarate de décyle, le laurate de stéaryle et le béhénilate de béhényle. On utilise de préférence le stéarate de cétyle.

Le composant iii) comprend un ou plusieurs agents émulsifiants permettant de former une dispersion des composants i) et ii) dans l'eau.

De préférence, le composant iii) est constitué d'un ou plusieurs agents émulsifiants non-ioniques et/ou amphotères. Il s'agit de combinaisons d'agents de surface non ioniques et/ou amphotères qui se distinguent par une partie lipophile, linéaire, alkyle, alkylène, alkylaryle et au minimum d'un groupe hydrophile. Cette fonction hydrophile peut être aussi bien un groupe ionique que non ionique.

Les agents émulsifiants non ioniques contiennent en tant que groupes hydrophiles, un groupe polyol, un groupe polyalkylèneglycoléther ou une combinaison de polyols et de groupes polyglycoléther

On donnera la préférence aux agents émulsifiants type H/E (huile dans l'eau) qui contiennent au minimum un des composés sélectionnés dans le groupe consistant en :
c1 : dérivés d'alcool linéaire en C8 - C24, acide gras en C12 - C22, alkyl C8 - C15 phénol, ou alkyl polyol, avec 2 à 50 moles d'oxyde d'éthylène et/ou 0 à 5 moles d'oxyde de propylène,
c2 : mono ou diester d'acides gras saturés et insaturés en C6 - C22 et de glycérol ou de sorbitol, éthoxylés ou non,
c3 : mono et oligoglucoside d'alkyl en C8 - C22 ou leurs analogues éthoxylés,
c4 : huile de ricin et huile de ricin hydrogénée avec 15 à 60 moles d'oxyde d'éthylène,
c5 : polyols, en particulier ester de polyglycérol, comme par exemple polyricinoléate de polyglycérol, ou poly-12-hydroxystéarate de polyglycérol,
et/ou les mélanges de ces composés.

Les agents émulsifiants amphotères sont du type bétaïne, tels que les dérivés d'imidazoline ou d'acides aminés de C2 à C18.

On peut utiliser comme agents émulsifiants amphotères, des dérivés de composés du type :
- N-alkyl-N,N-diméthyl glycinate d'ammonium, comme par exemple le diméthyl glycinate d'ammonium d'acide gras de coprah,
- N-acyl-aminopropyl-N, N-diméthyl-glycinate d'ammonium,
- 2-alkyl-3-carboxylméthyl-3-hydroxyéthylimidazoline où la chaîne alkyl comprend de 8 à 18 atomes de carbone, et
- cocosacylaminoéthylhydroxyéthylcarboxyméthylglycinate.

On pourra utiliser notamment les dérivés d'amides d'acides gras connus sous la désignation CFTA cocamidopropylbétaïne.

On peut utiliser des agents émulsifiants amphotères dérivés d'un groupe -alkyl- en C8 - C18 ou -acyl- et dont la molécule contient au moins un groupement amine libre et une fonction -COOH- ou -SO₃H-, par exemple des dérivés d'acides aminés de C2 à C18, tels que la N-alkylglycine, le N-alkylaminopropionate, le N-alkylsarcosinate et le N-alkyliminodipropionate.

On préfèrera les agents émulsifiants amphotères tels que les N-cocoalkylaminopropionates, les cocoacylaminoéthylaminopropionates et les acylsarcosinates en C12 - C18.

Le composant iii) devient facultatif si on veut réaliser, par exemple par des moyens mécaniques, une dispersion des composants i) et iii) dans l'eau.

Les alcools gras, esters cireux et agents émulsifiants sont choisis de manière à ne pas engendrer de problème d'odeur au niveau de la composition de la lotion.

D'autres composants émollients secondaires (alcools gras et esters de plus courte chaîne, etc.) peuvent être envisagés en plus à condition qu'ils ne modifient pas les propriétés du composant aqueux émollient.

Le composant aqueux émollient b) comprend, en poids de matières actives, de 35 à 90 % du composant i), de 1 à 50 % du composant ii) et de 0 à 20 % du composant iii), et de 0 à 50 % de cire ou huile minérale iv), le total des quantités des composants i), ii), iii) et iv) s'élevant à environ 100 %. De préférence, on utilise de 1 à 7 et plus préférentiellement de 1,5 à 5 % en poids de matières actives d'agent émulsifiant iii).

Le composant émollient b) de la lotion est aqueux sous forme de dispersion ou suspension. De préférence, il est sous la forme d'une dispersion. On entend par dispersion au sens large, un mélange d'une phase liquide ou solide sous forme de globules ou particules dans une autre phase liquide servant de véhicule. Le composant émollient comprend de 1 à 50 % en poids de matières actives. Plus précisément, il comprend entre environ 15 à environ 45 pour cent en poids de matières actives et entre environ 55 à 85 pour cent en poids d'eau, et de préférence entre environ 20 et environ 40 pour cent en poids de matières actives et entre environ 60 et environ 80 pour cent en poids d'eau. Une partie de l'eau peut être substituée par une huile ou cire d'origine minérale, telle que de l'huile ou cire de paraffine. La concentration en huile ou cire d'origine minérale iv) dans le composant aqueux émollient, est alors d'environ 1 à 10 pour cent en poids de matières actives. Pour le traitement des produits papetiers absorbants, on préférera un composant émollient contenant peu d'eau en quantité.

Quelle que soit la composition du composant émollient b) défini dans la description qui précède, son état est liquide à une température d'au moins 5°C. De préférence, le composant émollient est liquide à température ambiante, c'est-à-dire entre environ 10 et environ 40°C.

Le composant aqueux émollient est biodégradable.

La dispersion aqueuse est préparée dans une cuve munie d'un dispositif de mélange, d'un système de refroidissement et d'un échangeur de température. Le mélange ainsi obtenu passe dans un homogénéisateur. La dispersion est stable chimiquement et physiquement. Elle est homogène. Elle ne se sépare et n'épaissit quasiment pas. La dispersion est un agent émollient pour la peau. Un composé du type alkyl ester d'acide gras en tant que tel est connu pour avoir comme fonction de lubrifier la peau et d'éviter les pertes par évaporation de l'humidité contenue dans la peau afin d'empêcher tout dessèchement de la peau. Un composé du type alcool gras est quant à lui connu pour avoir comme fonction d'adoucir et de rendre lisse la surface de la peau.

Des exemples plus précis du composant aqueux émollient sont donnés ci-après.

| COMPOSANT 1 | COMPOSANT 2 |
|---|---|
| a) Alcools gras linéaires, saturés C18-C24 | a) Alcools gras linéaires, saturés C18-C22 |
| b) Esters cireux linéaires, saturés C32 | b) Esters cireux linéaires, saturés C28 |
| c) Agent émulsifiant : alcools gras éthoxylés | c) Agent émulsifiant : alcools gras éthoxylés |

Le composant c) est un solvant du type polyol, huile minérale ou leurs mélanges. Par polyol, on entend ici par exemple un propylène glycol, un dipropylène glycol ou tout composé équivalent. L'huile minérale est du type huile de vaseline blanche, par exemple les huiles minérales commercialisées sous le nom MARCOL ou PRIMOL par la société ESSO.

D'autres additifs secondaires peuvent, le cas échéant, être ajoutés à la composition de la lotion. Il s'agit d'agents classiquement utilisés pour les lotions, les crèmes ou tout produit émollient. On peut citer, par exemple, des épaississants, des parfums, du menthol, de l'eucalyptus, du niaouli, ou encore des composés bactéricides, virucides, etc. Ces agents sont ajoutés à la lotion dans des quantités appropriées.

Des composants ayant des propriétés calmantes ou cicatrisantes pour les irritations de la peau, notamment du nez, peuvent être incorporés dans la lotion. Des exemples de tels composants sont l'allantoïne et certains extraits végétaux.

La préparation de la lotion se fait par mélange sous agitation, des composants a), b) et c).

La composition de la lotion, dans le cas où l'ensemble des trois constituants est présent, comprend de 1 à 10 pour cent en poids du composant a) contenant essentiellement le composé ammonium quaternaire, de 5 à 99 pour cent en poids du composant aqueux émollient b) et q.s.p. 100 pour cent en poids du solvant c).

De préférence, elle comprend de 1 à 6 pour cent en poids du composant a), de 10 à 60 pour cent en poids du composant aqueux émollient b) et q.s.p. 100 pour cent en poids du solvant c).

Une autre composition pour la lotion peut comprendre le composant aqueux émollient b) et un composant c) à titre de solvant, les composants b) et c) sont alors simplement mélangés, le composant b) étant dispersé dans le composant ou solvant c). Ce type de lotion comprend de 5 à 100 pour cent en poids du composant aqueux émollient b) et q.s.p. 100 pour cent en poids du composant c).

Quelle que soit la composition de la lotion définie dans la description qui précède, son état est liquide à une température d'au moins 5°C. De préférence, la composition est liquide à température ambiante, c'est-à-dire entre environ 10 et environ 40°C, ce qui va permettre de l'appliquer directement sur un produit en papier absorbant, par des moyens classiques. Elle est de plus stable et homogène. Aucune séparation des phases ou phénomène de décantation n'apparaît. Sa viscosité est appropriée pour être appliquée par des moyens classiques : pulvérisation, enduction, etc, sur la surface d'un produit en papier absorbant. Elle peut être préparée à l'avance avant d'être appliquée sur le produit ou la feuille, ce qui évite des installations pour sa préparation sur le site industriel où a lieu l'application de la lotion sur le produit.

La composition de la lotion ainsi formulée appliquée sur un produit papetier absorbant, adoucit en surface ce produit. La lotion a pour principal effet, d'une part, de donner au papier un toucher doux et glissant tout en restant sec et, d'autre part, d'adoucir la surface de la peau au contact de ce papier. Dans le cas par exemple des mouchoirs en papier, la composition adoucissante et émolliente confère un toucher notablement doux aux mouchoirs et permet de diminuer nettement les phénomènes d'irritation rencontrés chez les personnes se mouchant fréquemment avec des mouchoirs classiques.

On décrira ci-après plus en détails l'application de la lotion aux produits papetiers et plus particulièrement à une feuille en ouate de cellulose qui sera transformée pour la fabrication de mouchoirs en papier. Les produits papetiers peuvent être, en totalité ou partiellement, traités ou imprégnés par la lotion.

La feuille en ouate de cellulose destinée à être traitée, est obtenue par tout procédé classique de fabrication d'ouate de cellulose. Les pâtes utilisées sont classiques. Elles peuvent être des pâtes vierges du type chimique et/ou CTMP chimicothermomécaniques blanchies, de feuillus et/ou de résineux, des pâtes désencrées de feuillus et/ou de résineux ou leurs mélanges. La composition de fabrication ou suspension aqueuse de fibres comprend par exemple un mélange de 60 % de pâte chimique blanchie de résineux et 40 % de pâte chimique blanchie d'eucalyptus. Dans le cas d'une utilisation de fibres recyclées, la composition de fabrication comprend par exemple de 50 à 95 % en poids de pâte désencrée.

La feuille en ouate de cellulose destinée à être traitée, est obtenue par tout procédé classique de fabrication d'ouate de cellulose. On peut incorporer comme additif dans la phase humide de fabrication de la feuille, un agent résistant humide. La feuille est crêpée ou non. Dans une application pour les mouchoirs, la feuille peut être calandrée ou non. Elle peut également être stratifiée ou non. Les strates ou couches peuvent être de composition fibreuse et/ou chimique identique ou différente. La feuille est composée d'un ou plusieurs plis, et de préférence de deux ou trois plis.

Un mode de réalisation préféré de la fabrication des mouchoirs consiste à fabriquer un mouchoir comprenant trois plis de nature ou composition différente. Le pli central a une composition fibreuse essentiellement à base de fibres longues, par exemple des pâtes à base de fibres de résineux, de préférence pins et épicéas. On incorpore dans la composition de fabrication de ce pli, un additif résistant humide. Si cet additif est déjà présent dans une certaine quantité dans chacun des deux autres plis, on incorpore une quantité plus importante de celui-ci dans le pli central. Cet additif est par exemple une résine résistante humide du type polyamide épichlorhydrine, commercialisée sous le nom KYMENE SLX par la société HERCULES. Les deux autre plis sont placés de part et d'autre du pli central et forment les surfaces de la feuille. Ils ont une composition fibreuse essentiellement à base de fibres courtes, par exemple des pâtes à base de fibres d'eucalyptus. Ces plis comprennent un adoucissant ou déliant. La feuille ainsi constituée présente une très bonne résistance humide et donc une bonne solidité du fait en particulier de la composition du pli central. Par ailleurs, elle offre une douceur de surface améliorée par le choix de la composition fibreuse et chimique des deux autres plis externes.

Le grammage de la feuille varie dans l'intervalle allant d'environ 12 à 65 g/m². Pour un mouchoir de type facial correspondant aux mouchoirs en boite dans le commerce, le grammage est d'environ 30 à 45 g/m², et pour un mouchoir plié et conditionné en étui, le grammage est d'environ de 35 à 65 g/m².

La lotion est appliquée sur au moins une face d'une feuille en ouate de cellulose à l'état sec et de préférence sur les deux faces externes de la feuille. Ce traitement peut être effectué à plusieurs stades de la fabrication de la feuille, dès que celle-ci a été séchée. Sur une machine à papier classique, il peut avoir lieu directement après l'étape de séchage de la feuille sur le cylindre Yankee, une fois que la feuille est crêpée ou après l'étape de séchage par soufflage traversant pour un autre procédé de fabrication. A ce stade, un seul pli est traité sur une seule face. Le traitement peut encore avoir lieu à l'étape de rebobinage, lorsque plusieurs plis sont associés pour former la feuille. Une ou les deux faces externes de la feuille sont traitées, successivement ou simultanément. L'application de la lotion peut aussi être réalisée dans la phase de transformation de la feuille en produit fini, papier toilette, mouchoir, etc. Dans le cas de la fabrication des mouchoirs, les deux faces de la feuille sont traitées par exemple juste avant l'étape de gaufrage des bords délimitant un mouchoir, étape qui a lieu avant les étapes de découpe et de pliage du mouchoir (un procédé de gaufrage des bords est décrit dans le brevet français n° 2 698 314) ou encore par exemple après gaufrage et association des plis. Le produit peut n'être gaufré que sur une seule de ses faces. Il peut l'être encore sur tout ou partie de ses surfaces. Toute transformation autre qu'un gaufrage et destinée à donner un aspect ou un motif spécifique à la feuille de papier peut aussi être envisagée. Un traitement par lotion du produit fini en papier absorbant, peut également être prévu. On a observé de manière surprenante que les surfaces d'une feuille, traitées et donc imprégnées de la lotion à quel que niveau que ce soit du procédé de fabrication ou de transformation de la feuille (après séchage de cette dernière), se gaufraient sans problème. Ceci est un avantage par rapport à des lotions de l'art antérieur, en particulier certaines lotions présentant un toucher gras, qui ne pouvaient pas être appliquées sur des surfaces du papier qui allaient être gaufrées par la suite, empêchant le gaufrage de s'effectuer sur le papier.

La lotion est appliquée sur le produit ou la feuille dans une quantité allant d'environ 0,30 à environ 20 et de préférence d'environ 0,65 à environ 15 pour cent en poids (poids de la composition aqueuse) sur la base du poids sec du produit (avant application de la lotion). De préférence, elle est appliquée dans une quantité allant d'environ 1 à environ 10 pour cent en poids de fibres sèches. Cela revient à appliquer sur chaque face du produit ou de la feuille, une quantité variant d'environ 0,3 g/m² à environ 3 g/m².

Plus préférentiellement, en optimisant les quantités de lotion utilisées et la douceur de surface du papier recherchée, on applique des quantités de lotion inférieures à environ 2 % en poids de matières actives sur la base du poids sec du produit. Le produit fini ainsi traité comprend alors une quantité inférieure à 2 % en poids de matières actives de lotion, sur la base du poids sec du produit papetier absorbant.

De manière générale, on obtient une douceur de surface nettement améliorée en sélectionnant, pour la composition de la lotion, au moins un ester cireux ayant au total de 24 à 48 atomes de carbone et en l'incorporant dans la composition dans une quantité suffisante de manière à ce qu'au moins 3 % en poids de matières actives de cet ester, sur la surface du poids sec du produit papetier absorbant, se retrouvent en surface du produit papetier absorbant.

La lotion peut être appliquée par différents moyens tels que la pulvérisation, l'enduction, l'impression comme la flexographie, ou tout autre technique permettant de déposer en surface de la feuille, la lotion.

La pulvérisation est effectuée par un système de buses classiques, des gouttelettes de la lotion sont projetées sur une face externe de la feuille. Elle est aussi réalisée par des dispositifs à mélange d'air ou sans air et sous basse pression ou encore au moyen de rotors. Ce procédé est simple d'application et la feuille n'est pas en contact avec le dispositif d'application. Avec une application par pulvérisation, on a remarqué qu'en traitant les deux faces externes d'une feuille comprenant trois plis, la lotion pénétrait en partie à l'intérieur de la feuille au niveau du pli interne. Suivant cette technique, on applique des quantités d'environ 1,5 g/m² à 3 g/m² par face.

L'enduction se fait au moyen d'un cylindre tramé sur lequel est déposée la lotion. Le cylindre est mis en contact avec une face de la feuille. Les deux faces de la feuille peuvent être traitées simultanément, un cylindre par face. La lotion peut être déposée sur toute la surface du cylindre ou partiellement, par exemple sous forme de bandes. On pourrait également envisager de déposer des quantités variables de lotion sur des parties de surface du cylindre et par voie de conséquence sur la face de la feuille. En utilisant ce procédé d'enduction, on a remarqué que la lotion ne pénétrait pas à l'intérieur de la feuille comprenant trois plis, au niveau du pli interne, et restait bien localisée en surface de la feuille. De ce fait, on a pu réduire avantageusement les quantités appliquées de la lotion selon l'invention, sur chaque face de la feuille. Les quantités appliquées par la technique d'enduction varient dans l'intervalle allant d'environ 0,3 g/m² à environ 2 g/m² par face et de préférence d'environ 0,5 g/m² à environ 1,5 g/m² par face. D'autres techniques d'enduction appropriées sont envisageables.

Des essais sur machine pilote ont été effectués en utilisant différentes compositions pour la lotion. Une feuille comprenant trois plis est traitée sur les deux faces. Le traitement se fait soit par pulvérisation par un dispositif muni de rotors, soit au moyen d'un cylindre d'enduction.

### Témoin 0

Une feuille en ouate de cellulose comprenant trois plis de 18 g/m² chacun, est transformée en mouchoirs découpés, dont les bords sont collés et gaufrés suivant le procédé décrit dans le brevet français N° 2 698 314.

### Exemple 1 (en dehors de l'invention)

On utilise une composition comprenant 100 pour cent en poids du composant aqueux émollient, correspondant à la lotion A dont la composition est la suivante.

| Lotion A (dispersion) | |
|---|---|
| i) | Alcools gras linéaires, saturés C18-C24 |
| ii) | Esters cireux linéaires, saturés C32 |
| iii) | Agent émulsifiant alcools gras éthoxylés |

Cette lotion est appliquée sur une feuille en ouate de cellulose par le dispositif de pulvérisation muni de rotors, sur la machine pilote. La feuille en ouate de cellulose à l'état sec, destinée à être traitée, comprend trois plis de 17 g/m² chacun et est calandrée. La lotion est appliquée dans une quantité de 2,5 g/m² par face. La feuille ainsi traitée au moyen de la lotion A, est ensuite transformée en mouchoir suivant le procédé décrit dans le brevet français N° 2 698 314.

La même feuille qui n'a pas été traitée par la lotion, sera également transformée en mouchoirselon le procédé mentionné ci-dessus et servira de témoin 1.

### Exemple 2 (en dehors de l'invention)

La composition de la lotion A de l'exemple 1 est appliquée sur une feuille calandrée comprenant trois plis de 20,5 g/m² chacun. Le traitement se fait au moyen d'un cylindre d'enduction. On applique une quantité de 0,6 g/m² sur chaque face de la feuille si bien que la quantité totale appliquée est relativement faible, ce qui est particulièrement avantageux. La feuille ainsi traitée au moyen de la lotion A est ensuite transformée en mouchoir suivant le procédé décrit dans le brevet français N° 2 698 314.

La même feuille qui n'a pas été traitée par la lotion, sera également transformée en mouchoir selon le procédé mentionné ci-dessus et servira de témoin 2.

### Exemple 3

On prépare une lotion B à base des trois composants a), b) et c). La composition de cette lotion B comprend :

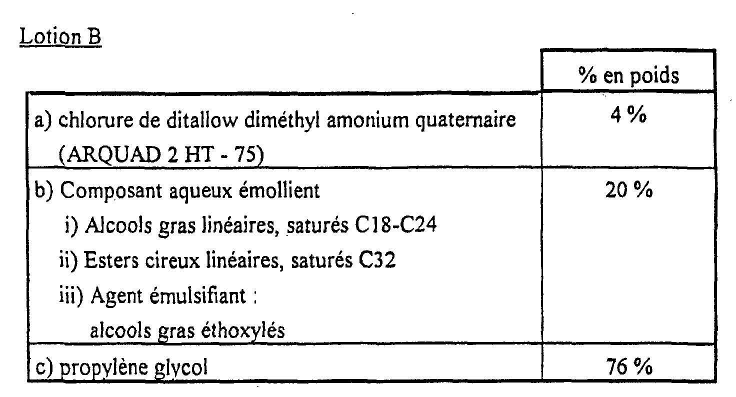

La lotion B est appliquée par le dispositif de pulvérisation muni de rotors. La feuille en ouate de cellulose destinée à être traitée comprend trois plis de 18 g/m² chacun et est calandrée. La quantité appliquée sur chaque face est de 2,5 g/m². La feuille ainsi traitée au moyen de la lotion B est ensuite transformée en mouchoir suivant le procédé décrit dans le brevet français N° 2 698 314.

La même feuille qui n'a pas été traitée par la lotion, sera également transformée en mouchoir selon le procédé mentionné ci-dessus et servira de témoin 3.

### Exemple 4

Une lotion C est préparée. Sa composition est la suivante :

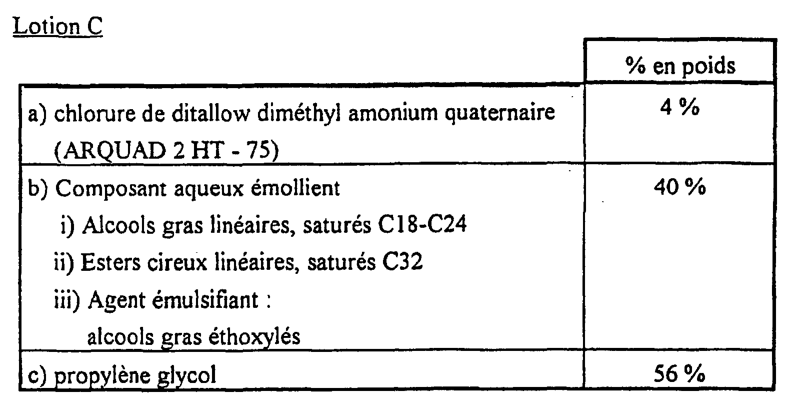

Elle est appliquée sur la feuille de papier au moyen d'un cylindre d'enduction. La feuille en ouate de cellulose qui va être traitée comprend trois plis de 20,5 g/m² chacune et est calandrée. La quantité appliquée est de 0,6 g/m². La feuille ainsi traitée au moyen de la lotion C est ensuite transformée en mouchoir suivant le procédé décrit dans le brevet français N° 2 698 314.

La même feuille qui n'a pas été traitée par la lotion, correspond au témoin 2.

### Exemple 5

La composition de la lotion C est utilisée pour traiter une feuille similaire à celle de l'exemple 4 sur la machine pilote par enduction. Seule la quantité de lotion appliquée varie. La quantité par face est ici de 1,3 g/m². La feuille ainsi traitée au moyen de la lotion C est ensuite transformée en mouchoir suivant le procédé décrit dans le brevet français N°2698314.

La même feuille qui n'a pas été traitée par la lotion, correspond au témoin 2.

Les mouchoirs issus des exemples 1 à 5, et les mouchoirs témoins ont fait l'objet de tests sensoriels réalisés sur 40 personnes.

Une série de test a été effectué sur les paramètres de douceur, de souplesse et d'épaisseur. La personne qui teste le produit, choisit un qualificatif sur une échelle verbale. La méthode consiste à attribuer des notes à cette échelle verbale suivant le tableau de correspondance ci-après, en comparant pour un paramètre donné, d'une part, un des mouchoirs d'un exemple 1 à 5, et, d'autre part, le mouchoir témoin correspondant 1 à 5, avec le même témoin 0 :

| Echelle verbale/Note | |
|---|---|
| nettement moins | -3 |
| moins | -2 |
| probablement moins | -1 |
| pas de différence | 0 |
| probablement plus | +1 |
| plus | +2 |
| nettement plus | +3 |

On multiplie le nombre de personnes ayant choisi un qualificatif donné par la note correspondant à ce qualificatif. Puis on fait la somme des points obtenus que l'on divise par le nombre total de personnes pour obtenir une note moyenne. Cette note moyenne située entre -3 et +3, donne le résultat du test. Les résultats sont rapportés dans le tableau I qui suit. Il faut remarquer que les témoins 1 à 5 sans lotion présentent déjà un niveau assez élevé de douceur, propre au procédé particulier de fabrication de la feuille en ouate de cellulose. C'est donc par rapport à un niveau reconnu comme bon, que la douceur des mouchoirs imprégnés de la lotion selon l'invention, s'apprécie.

**TABLEAU I**

| | DOUCEUR | SOUPLESSE | EPAISSEUR |
|---|---|---|---|
| Témoin 1 | + 0,15 | - 0,6 | - 0,6 |
| Exemple 1 | + 1,2 | + 0, 2 | - 0,45 |
| Témoin 2 | + 0,35 | - 0,3 | - 0,15 |
| Exemple 2 | + 1,15 | + 0,1 | - 0,25 |
| Témoin 3 | + 1 | - 0,45 | - 0,25 |
| Exemple 3 | + 1,65 | - 0,35 | - 0,25 |
| Témoin 2 | + 0,55 | - 0,35 | - 0,1 |
| Exemple 4 | + 1,45 | - 0,15 | 0 |
| Témoin 2 | + 0,75 | - 0,35 | - 0,2 |
| Exemple 5 | + 2 | +0,05 | + 0,1 |

La significativité des résultats est calculée par la méthode du χ².

Les résultats de l'exemple 1 et du témoin 1 sont significatifs à 1 % concernant la souplesse et la douceur. Le résultat concernant l'épaisseur pour le témoin 1 est significatif à 1 %, le résultat concernant l'épaisseur de l'exemple lest significatif à 5 %.

Les résultats de l'exemple 3 et du témoin 3 sont significatifs à I % pour la douceur.

Il n'y a aucune significativité au niveau des résultats sur la souplesse et l'épaisseur concernant les exemples 2, 4 et 5 et leur témoin 2 respectif

Par contre, pour les mêmes exemples 2, 4 et 5 et témoin 2 respectif, les résultats sont significatifs à 1 % concernant la douceur.

Nous ne discuterons que les paramètres dont les résultats sont significatifs. L'épaisseur est perçue comme n'étant pratiquement pas modifiée.

La souplesse lorsque les résultats sont significatifs, est améliorée.

Enfin, pour quasiment l'ensemble des résultats significatifs des exemples 1 à 5, les mouchoirs sont perçus comme étant probablement plus à plus doux (selon l'échelle verbale) par rapport au mouchoir témoin 0 déjà d'une qualité douce.

La différence entre l'exemple et son témoin permet d'évaluer l'influence seule de l'application de la lotion sur la feuille en éliminant l'influence de la feuille et de sa fabrication.

Les meilleurs résultats (différence entre l'exemple 1 et son témoin) au niveau de la douceur ressortent pour les exemples 5 et 1, correspondant respectivement à la lotion C appliquée par enduction, dans une quantité de 1,3 g/m² par face et à la lotion A appliquée par pulvérisation, dans une quantité de 2,5 g/m² par face.

Relativement à la quantité appliquée, ce sont les résultats des mouchoirs des exemples 5, 4 et 2 (différence entre l'exemple et son témoin) qui sont les plus intéressants, c'est-à-dire pour les exemples 5 et 4, de la lotion C appliquée par enduction dans une quantité de 0,6 et 1,3 g/m² par face et pour l'exemple 2, de la lotion A appliquée par enduction dans une quantité de 0,6 g/m² par face.

Une hiérarchisation de certains mouchoirs sur le paramètre de douceur et sur la préférence globale sur l'ensemble des paramètres de douceur, souplesse et épaisseur, a également été effectuée. La méthode utilisée pour réaliser cette hiérarchisation est celle du rang moyen. Les résultats sont rapportés dans le tableau II ci-après.

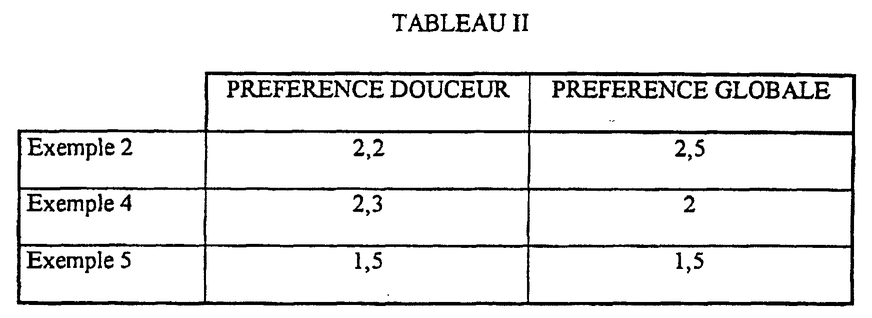

Les mouchoirs des exemples 2, 4 et 5 illustrent le procédé d'application par enduction.

Le mouchoir de l'exemple 5 ressort nettement en première position, par ordre de préférence, aussi bien sur le plan de la douceur que sur l'ensemble des paramètres.

Les mouchoirs des exemples 2 et 4 sont quasiment ex aequo en seconde position pour le paramètre de douceur.

Le mouchoir de l'exemple 4 vient en seconde position pour la préférence globale et le mouchoir de l'exemple 2 est en troisième position.

Par conséquent, la lotion C (exemples 4 et 5) est généralement préférée par rapport à la lotion A (exemple 2) et plus nettement lorsqu'elle est appliquée en ce qui concerne le procédé d'enduction, dans une quantité de 1,3 g/m² par face.

Concernant les propriétés physiques et mécaniques des mouchoirs ainsi imprégnés, on a mesuré les résistances sèches en sens marche (SM) et en sens travers (ST), l'allongement en sens marche (SM) et les résistances humides en sens marche (SM) et en sens travers (ST).

Les résultats pour les exemples 4 et 5 sont rapportés dans le Tableau III qui suit.

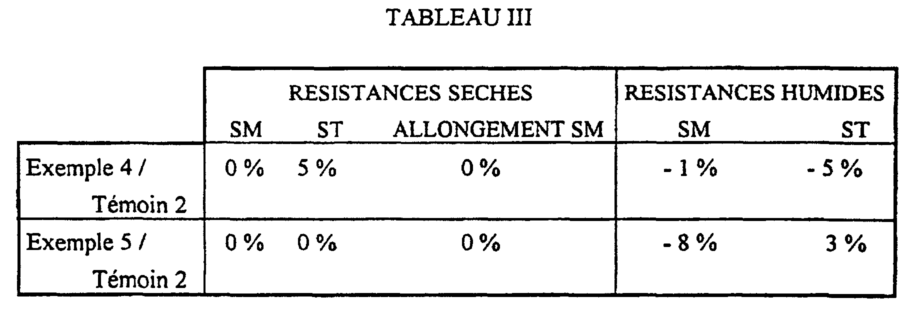

Les propriétés illustrées par les résultats du Tableau III ne sont donc pas modifiées de manière susbstantielle. Elles sont aussi bonnes que celles du témoin (une différence de 5 à 8 % n'étant pas significative pour les résistances humides en particulier). Ceci est un avantage appréciable.

Les propriétés d'absorption telles que la capacité d'absorption ont également été mesurées pour les mouchoirs de l'ensemble des exemples. On s'attendait à une perte importante de ces propriétés d'absorption Or, il s'avère que cette perte est tout à fait faible.

Le traitement des produits en papier absorbant par les lotions selon l'invention n'a donc pas de conséquences négatives sur les propriétés physiques et mécaniques du produit.

## Revendications

1. Composition pour une lotion adoucissante, liquide à une température d'au moins environ 5°C, destinée au traitement d'un produit papetier absorbant, **caractérisée en ce qu'**elle comprend :
a) de 1 à 10 pour cent en poids d'un composant contenant essentiellement un composé ammonium quaternaire,
b) de 5 à 99 pour cent en poids d'un composant aqueux émollient comprenant comme matière active :
i) un ou plusieurs alcools gras linéaires, saturés, ayant au moins 16 atomes de carbone, et
ii) un ou plusieurs esters cireux ayant au total au moins 24 atomes de carbone, et éventuellement
iii) un ou plusieurs agents émulsifiants non-ioniques et/ou amphotères, et
c) q.s.p 100 pour cent en poids d'un solvant du type polyol, huile minérale ou leurs mélanges.

2. Composition selon la revendication 1, **caractérisée en ce que** le composant aqueux émollient précité est une dispersion aqueuse.

3. Composition selon l'une quelconque des revendications précédentes, **caractérisée en ce que** le ou les alcools gras précités i) ont de 16 à 28 atomes de carbone, et le ou les esters cireux précités ii) ont de 24 à 48 atomes de carbone.

4. Composition selon l'une quelconque des revendications précédentes, **caractérisée en ce que** le ou les esters cireux précités ii) sont notamment synthétiques et sont dérivés d'acides gras linéaires, saturés, ayant de 6 à 24 atomes de carbone et d'alcools gras linéaires, saturés, ayant de 6 à 24 atomes de carbone.

5. Composition selon l'une quelconque des revendications précédentes, **caractérisée en ce que** le composant aqueux émollient précité comprend de 1 à 50 % en poids de matières actives.

6. Composition selon la revendication 5, **caractérisée en ce que** le composant aqueux émollient précité comprend de 15 à 45 pour cent en poids de matières actives et de 55 à 85 pour cent en poids d'eau et de préférence, de 20 à 40 pour cent en poids de matières actives et de 60 à 80 pour cent en poids d'eau.

7. Composition selon l'une quelconque des revendications précédentes, **caractérisée en ce que** le composant aqueux émollient précité comprend en poids de matières actives
i) 35 à 90 % d'alcools gras linéaires saturés ayant de 18 à 24 atomes de carbone,
ii) 1 à 50 % d'esters cireux ayant au total de 24 à 48 atomes de carbone,
iii) 0 à 20 % d'agents émulsifiants non-ioniques et/ou amphotères, et
iv) 0 à 50 % de cire ou huile minérale,
le total des quantités en composants s'élevant à 100 % en poids de matières actives.

8. Composition selon l'une quelconque des revendications précédentes, **caractérisée en ce que** le composant aqueux émollient précité comprend l'alcool béhénique comme alcool gras i).

9. Composition selon l'une quelconque des revendications précédentes, **caractérisée en ce qu'**elle comprend
a) de 1 à 6 pour cent en poids du composant contenant essentiellement un composé ammonium quaternaire,
b) de 10 à 60 pour cent en poids du composant aqueux émollient précité, et
c) q.s.p 100 pour cent en poids du solvant précité.

10. Composition selon l'une quelconque des revendications précédentes, **caractérisée en ce que** le composé ammonium quaternaire précité est un sel de formule (I) suivante 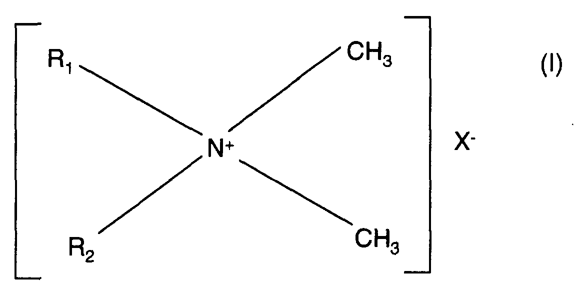 où R₁ est un groupement aliphatique ayant de préférence de 12 à 18 atomes de carbone, R₂ est un groupement aliphatique ayant de préférence de 12 à 18 atomes de carbone ou un groupement aryle, de préférence benzyle, et X est un halogène de type chlore.

11. Composition selon l'une quelconque des revendications précédentes, **caractérisée en ce que** le composant a) est un chlorure de ditallow diméthyl ammonium.

12. Composition selon l'une des revendications 1 à 11, **caractérisée en ce que** le composant a) est un mélange comprenant un chlorure d'alkyl benzyl diméthyl ammonium et un alcool gras éthoxylé.

13. Composition selon l'une des revendications 1 à 10, **caractérisée en ce que** le composé ammonium quaternaire précité est un mono-, di- ou tri-esters ammonium quaternaire de formule (II) suivante 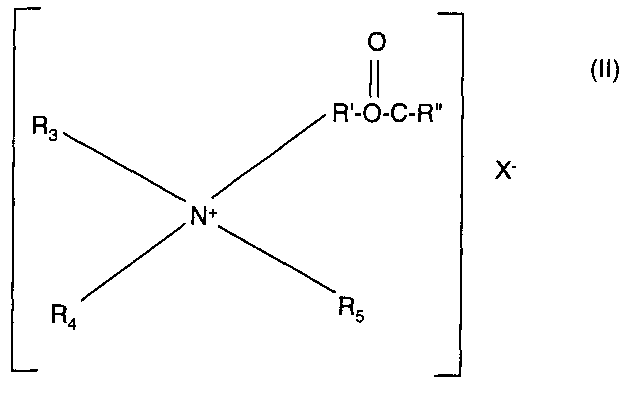 dans laquelle
R' est un groupement alkyle ayant de 1 à 6 atomes de carbone,
R" est un groupement alkyle ayant de 12 à 18 atomes de carbone,
R₃ est un groupement alkyle ayant de 1 à 6 atomes de carbone, et de préférence méthyle,
R₄ est un groupement alkyle ayant de 1 à 6 atomes de carbone, un groupement hydroxy-alkyle de 1 à 6 atomes de carbone ou un groupement 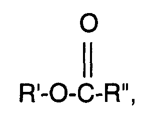 et
R₅ un groupement alkyle de 1 à 6 atomes de carbone, un groupement hydroxy-alkyle de 1 à 6 atomes de carbone ou un groupement 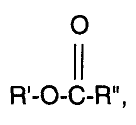 et
X⁻ est un anion, tel qu'un halogénure du type chlorure ou un méthyl sulfate.

14. Composition selon la revendication 13, **caractérisée en ce que** R' est un groupement éthylène, R" est un groupement alkyle ayant de 16 à 18 atomes de carbone du type tallow, R₃ est un groupement méthyle, R₄ est un groupement hydroxy- éthylène, R₅ est un groupement 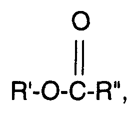 et X⁻ est un méthyl sulfate.

15. Composition selon l'une quelconque des revendications précédentes, **caractérisée en ce que** le solvant précité est un propylène glycol ou un dipropylène glycol.

16. Utilisation d'une lotion adoucissante pour le traitement d'un produit papetier absorbant, **caractérisée en ce que** la composition de ladite lotion est définie selon l'une des revendications 1 à 15 et **en ce qu'**une quantité d'environ 0,30 à environ 20 pour cent en poids sur la base du poids sec du produit précité, de ladite lotion, est appliquée sur le produit.

17. Utilisation selon la revendication 16, **caractérisée en ce qu'**une quantité d'environ 1 à environ 10 pour cent en poids, sur la base du poids sec du produit précité, de la lotion précitée, est appliquée sur ledit produit.

18. Produit papetier absorbant, **caractérisé en ce qu'**au moins une surface dudit produit est imprégnée d'une lotion dont la composition est définie selon l'une des revendications 1 à 15.

19. Produit papetier absorbant, **caractérisé en ce qu'**au moins une surface dudit produit est imprégnée d'une lotion,
**en ce qu'**il comprend :
- des fibres papetières,
- au moins un composé ammonium quaternaire,
- au moins un alcool gras linéaire saturé, ayant au moins 16 atomes de carbone, et
- au moins un ester cireux ayant de 24 à 48 atomes de carbone, et
**en ce qu'au** moins 3 pour cent en poids de matières actives de l'ester cireux, sur la base du poids sec du produit papetier absorbant, se retrouvent en surface du produit papetier absorbant.

20. Produit selon la revendication 18 ou 19, **caractérisé en ce qu'**il est un mouchoir en papier jetable.

## Patentansprüche

1. Zusammensetzung für eine weichmachende, bei einer Temperatur von mindestens ungefähr 5°C flüssige, zur Behandlung eines absorbierenden Papierprodukts bestimmte Lotion, **dadurch gekennzeichnet, dass** sie folgendes umfasst:
a) zwischen 1 bis 10 Gew.-% einer Komponente, die im Wesentlichen eine quartäre Ammoniumverbindung aufweist,
b) zwischen 5 bis 99 Gew.-% einer wässrigen weichmachenden Komponente, die folgenden Aktivstoff aufweist:
i) eine bzw. mehrere lineare gesättigte Fettalkohole mit mindestens 16 Kohlenstoffatomen, und
ii) eine bzw. mehrere wachsartige Ester mit mindestens insgesamt 24 Kohlenstoffatomen, und eventuell
iii) eine bzw. mehrere nicht-ionische und/oder amphotere emulgierende Wirkstoffe, und
c) eine ausreichende Menge Lösungsmittel vom Typ Polyol, Mineralöl oder ihre Mischungen zum Ergänzen auf insgesamt 100 Gew.-%.

2. Zusammensetzung nach Anspruch 1, **dadurch gekennzeichnet, dass** die zuvor genannte wässrige weichmachende Komponente eine wässrige Dispersion ist.

3. Zusammensetzung nach einem der vorangehenden Ansprüche, **dadurch gekennzeichnet, dass** der bzw. die zuvor genannte(n) Fettalkohol(e) i) 16 bis 28 Kohlenstoffatome haben und dass der bzw. die vorgenannte(n) wachsartige(n) Ester ii) 24 bis 48 Kohlenstoffatome haben.

4. Zusammensetzung nach einem der vorangehenden Ansprüche, **dadurch gekennzeichnet, dass** der bzw. die vorgenannte(n) wachsartige(n) Ester ii) insbesondere synthetisch sind und Derivate von gesättigten linearen Fettsäuren mit 6 bis 24 Kohlenstoffatomen sind und Derivate von gesättigten linearen Fettalkoholen mit 6 bis 24 Kohlenstoffatomen sind.

5. Zusammensetzung nach einem der vorangehenden Ansprüche, **dadurch gekennzeichnet, dass** die zuvor genannte wässrige weichmachende Komponente zwischen 1 und 50 Gew.-% Aktivstoffe aufweist.

6. Zusammensetzung nach Anspruch 5, **dadurch gekennzeichnet, dass** die zuvor genannte wässrige weichmachende Komponente zwischen 15 und 45 Gew.-% Aktivstoffe und zwischen 55 und 85 Gew.-% Wasser und vorzugsweise zwischen 20 und 40 Gew.-% Aktivstoffe und zwischen 60 und 80 Gew.-% Wasser aufweist.

7. Zusammensetzung nach einem der vorangehenden Ansprüche, **dadurch gekennzeichnet, dass** die zuvor genannte wässrige weichmachende Komponente folgende Gew.-% Aktivstoffe umfasst:
i) 35 bis 90% gesättigte lineare Fettalkohole mit 18 bis 24 Kohlenstoffatomen,
ii) 1 bis 50% wachsartige Ester mit insgesamt 24 bis 48 Kohlenstoffatomen,
iii) 0 bis 20% nicht-ionische und/oder amphotere emulgierende Wirkstoffe, und
iv) 0 bis 50% Wachs oder Mineralöl,
wobei sich die Gesamtheit der Komponentenmengen auf 100 Gew.-% Aktivstoffe beläuft.

8. Zusammensetzung nach einem der vorangehenden Ansprüche, **dadurch gekennzeichnet, dass** die zuvor genannte wässrige weichmachende Komponente Behenalkohol als Fettalkohol i) aufweist.

9. Zusammensetzung nach einem der vorangehenden Ansprüche, **dadurch gekennzeichnet, dass** sie folgendes umfasst:
a) zwischen 1 und 6 Gew.-% der Komponente, die im Wesentlichen eine quartäre Ammoniumverbindung aufweist,
b) zwischen 10 und 60 Gew.-% der wässrigen weichmachenden Komponente, und
c) eine ausreichende Menge des zuvor genannten Lösungsmittels zum Ergänzen auf 100 Gew.-%.

10. Zusammensetzung nach einem der vorangehenden Ansprüche, **dadurch gekennzeichnet, dass** die zuvor genannte quartäre Ammoniumverbindung ein Salz mit folgender Formel (I) ist: 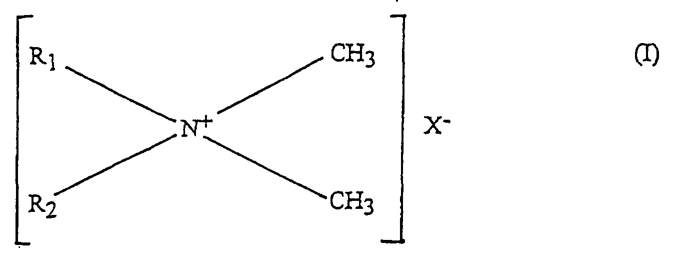 wobei R₁ eine aliphatische Gruppierung mit vorzugsweise 12 bis 18 Kohlenstoffatomen ist, R₂ eine aliphatische Gruppierung mit vorzugsweise 12 bis 18 Kohlenstoffatomen oder eine Arylgruppierung, vorzugsweise Benzyl, und X ein Halogen vom Chlortyp ist.

11. Zusammensetzung nach einem der vorangehenden Ansprüche, **dadurch gekennzeichnet, dass** die Komponente a) ein Ditallowdimethylammoniumchlorid ist.

12. Zusammensetzung nach einem der Ansprüche 1 bis 11, **dadurch gekennzeichnet, dass** die Komponente a) eine Mischung aus einem Alkylbenzyldimethylammoniumchlorid und einem ethoxyilierten Fettalkohol ist.

13. Zusammensetzung nach einem der Ansprüche 1 bis 10, **dadurch gekennzeichnet, dass** die zuvor genannte quartäre Ammoniumverbindung eine quartäre Mono-, Di- oder Triester-Ammoniumverbindung mit folgender Formel (II) ist: 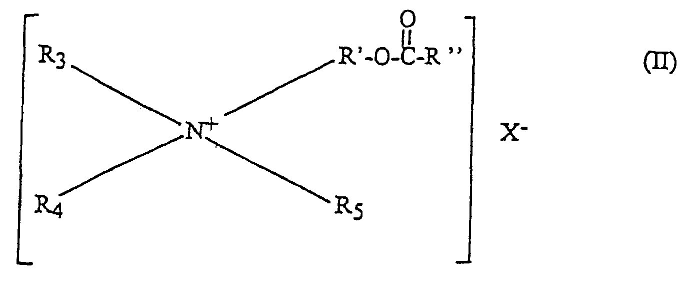 in der R' eine Alkylgruppierung mit 1 bis 6 Kohlenstoffatomen ist,
R" eine Alkylgruppierung mit 12 bis 18 Kohlenstoffatomen ist,
R₃ eine Alkylgruppierung mit 1 bis 6 Kohlenstoffatomen, vorzugsweise Methyl, ist,
R₄ eine Alkylgruppierung mit 1 bis 6 Kohlenstoffatomen, eine HydroxyAlkyl-Gruppierung mit 1 bis 6 Kohlenstoffatomen oder eine Gruppierung 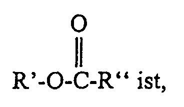 und
R₅ eine Alkylgruppierung von 1 bis 6 Kohlenstoffatomen, eine HydroxyAlkyl-Gruppierung mit 1 bis 6 Kohlenstoffatomen oder eine Gruppierung 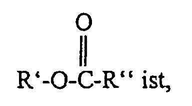 und
X⁻ ein Anion, wie z.B. ein Halogenid vom Typ Chlorid oder ein Methylsulfat ist.

14. Zusammensetzung nach Anspruch 13, **dadurch gekennzeichnet, dass** R' eine Gruppierung Ethylen ist, R" eine Alkylgruppierung mit 16 bis 18 Kohlenstoffatomen vom Typ Tallow ist, R₃ eine Methylgruppierung ist, R₄ eine Gruppierung Hydroxyethylen ist, R₅ eine Gruppierung 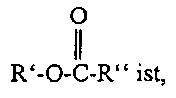 und
X⁻ ein Methylsulfat ist.

15. Zusammensetzung nach einem der vorangehenden Ansprüche, **dadurch gekennzeichnet, dass** das zuvor genannte Lösungsmittel ein Propylenglycol oder ein Dipropylenglycol ist.

16. Verwendung einer weichmachenden Lotion für die Behandlung eines absorbierenden Papierprodukts, **dadurch gekennzeichnet, dass** die Zusammensetzung der besagten Lotion definiert ist nach einem der Ansprüche 1 bis 15 und dass eine Menge von ungefähr 0,30 bis ungefähr 20 Gew.-% auf der Basis des Trockengewichts des zuvor genannten Produkts, der besagten Lotion, auf das Produkt aufgetragen wird.

17. Verwendung nach Anspruch 16, **dadurch gekennzeichnet, dass** eine Menge von ungefähr 1 bis ungefähr 10 Gew.-% auf der Basis des Trockengewichts des zuvor genannten Produkts, der zuvor genannten Lotion, auf das besagte Produkt aufgetragen wird.

18. Absorbierendes Papierprodukt, **dadurch gekennzeichnet, dass** mindestens eine Oberfläche des besagten Produkts mit einer Lotion imprägniert ist, deren Zusammensetzung nach einem der Ansprüche 1 bis 15 definiert ist.

19. Absorbierendes Papierprodukt, **dadurch gekennzeichnet, dass** mindestens eine Oberfläche des besagten Produkts mit einer Lotion imprägniert ist und dass es folgendes umfasst:
- Papierfasern,
- mindestens eine quartäre Ammoniumverbindung,
- mindestens einen gesättigten linearen Fettalkohol mit mindestens 16 Kohlenstoffatomen, und
- mindestens einen wachsartigen Ester mit 24 bis 48 Kohlenstoffatomen, und
**dadurch**, dass mindestens 3 Gew.-% Aktivstoffe des wachsartigen Ester, auf der Basis des Trockengewichts des absorbierenden Papierprodukts, sich an der Oberfläche des absorbierenden Papierprodukts wieder finden.

20. Produkt nach Anspruch 18 oder 19, **dadurch gekennzeichnet, dass** es ein Wegwerfpapiertaschentuch ist.

## Claims

1. A formulation for a softening lotion which is liquid at a temperature of at least about 5°C intended for the treatment of an absorbent paper product, **characterised in that** it contains:
a) 1 to 10 wt.% of a component containing substantially a quaternary ammonium compound,
b) 5 to 99 wt.% of an emollient aqueous component containing as active material:
i) one or more saturated linear fatty alcohols with at least 16 carbon atoms, and
ii) one or more waxy esters with a total of at least 24 carbon atoms, and optionally
iii) one or more non-ionic and/or amphoteric emulsifying agents, and
c) sufficient solvent of the polyol or mineral oil type or mixtures thereof to make the total up to 100 wt.%.

2. A formulation according to Claim 1, **characterised in that** the emollient aqueous component mentioned above is an aqueous dispersion.

3. A formulation according to one of the preceding Claims, **characterised in that** the fatty alcohol(s) i) mentioned above have 16 to 28 carbon atoms and the waxy ester(s) ii) mentioned above have 24 to 48 carbon atoms.

4. A formulation according to one of the preceding Claims, **characterised in that** the waxy ester(s) ii) mentioned above are in particular synthetic and are derived from saturated linear fatty acids with 6 to 24 carbon atoms and saturated linear fatty alcohols with 6 to 24 carbon atoms.

5. A formulation according to one of the preceding Claims, **characterised in that** the emollient aqueous component mentioned above contains 1 to 50 wt.% of active material.

6. A formulation according to Claim 5, **characterised in that** the emollient aqueous component mentioned above contains 15 to 45 wt.% of active material and 55 to 85 wt.% of water and preferably 20 to 40 wt.% of active material and 60 to 80 wt.% of water.

7. A formulation according to one of the preceding Claims, **characterised in that** the emollient aqueous component mentioned above contains, by weight, the following active materials:
i) 35 to 90 % of saturated linear fatty alcohols with 18 to 24 carbon atoms,
ii) 1 to 50 % waxy esters with a total of 24 to 48 carbon atoms
iii) 0 to 20 % non-ionic and/or amphoteric emulsifying agents, and
iv) 0 to 50 % wax or mineral oil,
the sum of the quantities of components amounting to 100 wt.% of active material.

8. A formulation according to one of the preceding Claims, **characterised in that** the emollient aqueous component mentioned above contains behenyl alcohol as fatty alcohol i).

9. A formulation according to one of the preceding Claims, **characterised in that** it contains:
a) 1 to 6 wt.% of the component containing substantially a quaternary ammonium compound,
b) 10 to 60 wt.% of the emollient aqueous component mentioned above, and
c) sufficient of the solvent mentioned above to make the total up to 100 wt.%.

10. A formulation according to one of the preceding Claims, **characterised in that** the quaternary ammonium compound mentioned above is a salt of the formula (I) below: 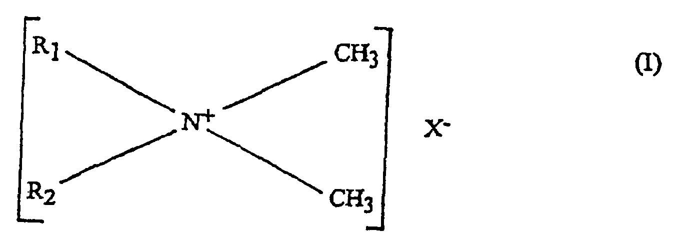 where R₁ is an aliphatic group with preferably 12 to 18 carbon atoms, R₂ is an aliphatic group with preferably 12 to 18 carbon atoms or an aryl group, preferably benzyl, and X is a halogen of the chlorine type.

11. A formulation according to one of the preceding Claims, **characterised in that** component a) is a ditallowdimethylammonium chloride.

12. A formulation according to one of Claims 1 to 11, **characterised in that** component a) is a mixture containing an alkylbenzyldimethylammonium chloride and an ethoxylated fatty alcohol.

13. A formulation according to one of Claims 1 to 10, **characterised in that** the quaternary ammonium compound mentioned above is a quaternary ammonium monoester, diester or triester of the formula (II) below 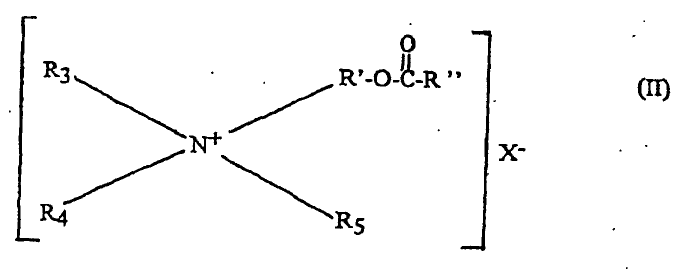 in which
R' is an alkyl group with 1 to 6 carbon atoms,
R" is an alkyl group with 12 to 18 carbon atoms,
R₃ is an alkyl group with 1 to 6 carbon atoms, preferably methyl,
R₄ is an alkyl group with 1 to 6 carbon atoms, a hydroxyalkyl group with 1 to 6 carbon atoms or a R'-O-C (=O) -R" group,
and
R₅ is an alkyl group with 1 to 6 carbon atoms, a hydroxyalkyl group with 1 to 6 carbon atoms or a R'-O-C (=O) -R" group,
and
X- is an anion, such as a halide of the chloride type or a methyl sulfate.

14. A formulation according Claim 13, **characterised in that** R' is an ethylene group, R" is an alkyl group with 16 to 18 carbon atoms of the tallow type, R₃ is a methyl group, R₄ is a hydroxyethylene group, R₅ is a R'-O-C(=O)-R" group and X⁻ is a methyl sulfate.

15. A formulation according to one of the preceding Claims, **characterised in that** the solvent mentioned above is a propylene glycol or a dipropylene glycol.

16. Use of a softening lotion to treat an absorbent paper product, **characterised in that** the formulation for said lotion is defined according to one of Claims 1 to 15 and that an amount of about 0.30 to about 20 wt.%, with respect to the dry weight of the product mentioned above, is applied to the product.

17. Use according to Claim 16, **characterised in that** an amount of about 1 to about 10 wt.%, with respect to the dry weight of the product mentioned above, of the lotion mentioned above, is applied to said product.

18. An absorbent paper product, **characterised in that** at least one face of said product is impregnated with a lotion the formulation for which is defined according to one of Claims 1 to 15.

19. An absorbent paper product, **characterised in that** at least one face of said product is impregnated with a lotion,
**in that** it contains:
- paper fibres,
- at least one quaternary ammonium compound,
- at least one saturated linear fatty alcohol with at least 16 carbon atoms, and
- at least one waxy ester with 24 to 48 carbon atoms, and
**in that** at least 3 wt.% of active waxy ester material, with respect to the dry weight of the absorbent paper product, is located at the surface of the absorbent paper product.

20. A product according to Claim 18 or 19, **characterised in that** it is a disposable paper handkerchief.
